# EUROPÄISCHE PATENTSCHRIFT

(11) **EP 0 494 904 B1**
(45) Veröffentlichungstag und Bekanntmachung des Hinweises auf die Patenterteilung: **15.09.1993**
(21) Anmeldenummer: 90914519.5
(22) Anmeldetag: 01.10.1990
(51) Int. Cl.: A61J 17/00, A61K 47/48

(54) **VORRICHTUNG ZUM ORALEN VERABREICHEN EINER WIRKSUBSTANZ ZUR PROPHYLAKTISCHEN BEHANDLUNG VON KARIES BEI SÄUGLINGEN**
DEVICE FOR ORAL ADMINISTRATION OF AN ACTIVE PRINCIPLE FOR PREVENTIVE TREATMENT OF CARIES IN BABIES
DISPOSITIF POUR L'ADMINISTRATION ORALE D'UN PRINCIPE ACTIF EN VUE DU TRAITEMENT PROPHYLACTIQUE DES CARIES CHEZ LES NOURRISSONS

(30) Priorität: 02.10.1989 CH 3577/89; 27.03.1990 DE 9003563 U
(43) Veröffentlichungstag der Anmeldung: 22.07.1992
(73) Patentinhaber: SUHONEN, Jouko, 8302 Kloten (CH)
(72) Erfinder: SUHONEN, Jouko, 8302 Kloten (CH)
(74) Vertreter: Richter, Werdermann & Gerbaulet
(86) Internationale Anmeldenummer: EP9001645
(87) Internationale Veröffentlichungsnummer: WO9104727

(56) Entgegenhaltungen:
- CH-A- 357 832
- DE-A- 3 503 777
- DE-C- 811 133
- US-A- 3 610 248

## Beschreibung

Die Erfindung ist im Bereich der Kleinkinderbetreuung und insbesondere im Bereich von Prophylaxemaßnahmen einsetzbar und betrifft eine Vorrichtung zum oralen Verabreichen einer Wirksubstanz zur prophylaktischen Behandlung von Karies bei Säuglingen, bestehend aus einem Sauger mit einer Mundscheibe und einem an dieser befestigten, lutschartigen Mundstück aus Gummi od.dgl. mit Durchbrechungen, durch die in Verbindung mit dem Speichel des Kindes die Wirksubstanz sich auflöst und austritt, wobei die Mundscheibe mit dem Mundstück lösbar verbunden ist, dessen Innenraum als Aufnahmekammer für die Wirksubstanz dient. Eine solche Vorrichtung ist aus der DE-C-811133 bekannt.

Die vorliegende Erfindung bezieht sich dabei auf eine Einrichtung für die Mundverabreichung einer Wirksubstanz über eine längere Zeit sowie auf einen Schnuller oder Nuggi für die Mundverabreichung einer Wirksubstanz über eine längere Zeit.

Die Zahnmedizin befaßt sich seit einiger Zeit vermehrt mit der sogenannten Prävention von Zahnkrankheiten, wie insbesondere von Karies, durch die Anwendung entsprechender Maßnahmen oder Therapien bereits bei Jugendlichen oder bei Kindern.

Bei mehreren Studien, die von verschiedenen Forschungsteams durchgeführt wurden, wurde festgestellt, daß Xylit für die Prävention von Karies geeignet ist.

Zusätzlich zu seiner wohldokumentierten Wirkung als nicht-kariogener Süßstoff konnte man für das Pentose-Derivat Xylit, wie bei S.Assev/G. Rolla, "Further studies on the growth inhibition of Streptococcus mutans OMZ 176 by xylitol", Acta.Path.Microbiol.Immunol.Scand.94:102,1986, beschrieben, auch einen kariostatischen Effekt nachweisen.

Neuere Forschungen haben ergeben, daß Xylit eine antibaketerielle oder vielmehr bakteriostatische Wirkung besitzt, da es die Glycolyse der Bakterien hemmt. Xylit wird wahrscheinlich von Bakterien aufgenommen, die jedoch nicht in der Lagz sind, dieses Monosaccharid als Energiequelle zu verwerten oder in Kohlenhydrat umzuwandeln. Xylit wird vom Fructose-PTS-System aufgenommen und phosphoryliert in diesem Prozeß. Xylitphosphat ist toxisch für die Bakterien und muß dephosphoryliert und ausgestoßen werden. Xylit verhindert den Stoffwechsel von Streptococcus mutans. Die Kombination von Xylit und Sorbit hat eine synergistische Hemmwirkung auf den Zuckermetabolismus von Streptococcus mutans. Xylit ist in Bezug auf Karieshemmung wohl das am besten erforschte der Monosaccharide. WHO-Xylitol-Feldstudien und Kaugummistudien bestätigen dies. Verwiesen sei auf die "Collaborative WHO xylitol field studies in Hungary - Three-year caries activity in institutionalized children", Acta.0dont.Scand.43:327-347, 1985, sowie auf P.Isokangas/J.Tiekso/P.Alanen/K.K.Mäkinen: "Longterm effext of xylitol chewing gum on dental caries", Community dent. Oral. Epidemiol. 17: 200-203,1989. Weitere Studien sind bekannt, die dieselben Resultate bestätigen.

In neueren Studien wurde weiter gezeigt, wie in Anwesenheit von Xylit das Streptococcus-mutans-Niveau generell, sowohl in Zahnplaque als auch im Speichel, abnimmt.

Verwiesen sei hier auf E. Söderling/K.K.Mäkinen/C.-Y. Chen/H.R. Pape Jr./W.Loesche/P.-L. Mäkinen: "Effect of sorbitol, xylitol und xylitol/sorbitol chewing gums an dental plaque", Caries Res. 23: 378-384,1989.

Daraus wird klar, daß in Anwesenheit von Xylit die Adhäsion der Streptococcus-mutans-Bakterien (Kariesinitiator-Bakterien) an der Zahnoberfläche abnimmt.

Diese Studien zeigen also deutlich, daß bereits eine Prävention gegen Karies im Kleinkindalter im Sinne einer Primärprävention wünschenswert wäre, was aber nicht unproblematisch ist. Da es sich gezeigt hat, daß die Verweildauer von Xylit in der Mundhöhle anscheinend eine wichtige Rolle spielt, muß der Wirkstoff durch geeignete Maßnahmen während einer längeren Zeit im Mund zurückgehalten werden.

Bei Kindern ab einem Alter von ca. drei oder vier Jahren ist die Abgabe von Xylit mittels Kaugummis, Bonbons und Schleckstengel möglich, mittels welchen Xylit nur relativ langsam während einer verlängerten Zeitperiode an die Mundhöhle abgegeben wird. Die 'Kariesprävention ist aber bereits ab dem Durchbruch des ersten Milchzahnes sinnvoll, um eine Streptococcus-mutans-Kolonisation durch Primärprävention zu verhindern und dadurch einen kariesfreien Zustand zu erreichen.

Die Abgabe von Kaugummis, Bonbons usw. ist aber bei Kleinkindern nicht möglich, da sie diese meistens unverzüglich verschlucken und so der Wirkstoff Xylit kaum oder gar nicht in der Mundhöhle verbleibt - von einer verlängerten'Verweilzeit ist hier gar nicht zu sprechen.

Dazu kommt im weiteren die Gefahr des Erstickens eines Kleinkindes.

Nach der DE-C-811133 ist ein Beruhigungsschnuller für Kleinkinder bekannt, der zu einem mit Medizin füllbaren Schnuller ausgestaltet ist, aus welchem die eingefüllte Medizin mit Süßigkeiten aller Art vermischt aus mehreren Sauglöchern des Saugers langsam vom Kinde herausgesaugt wird. Bei diesem Schnuller ist der Sauger mit einer Mundscheibe zu einem Saugerstück so vereinigt, daß ein Abziehen des Saugers möglich ist, um den Sauger mit der Medizin füllen zu können, wobei auch eine Befestigung des Saugers an dem Mundstück über eine Gewinderverbindung möglich ist. Durchbrechungen in dem Sauger ermöglichen eine Entnahme der Medizin während des Lutsch- und Saugvorganges, wobei die Durchbrechungen nicht gerichtet sind, um den Fluß der Medizin an ganz bestimmte Stellen des Mundes oder gezielt an das Zahnfleisch zu leiten, denn das Ziel, das mit diesem Schnuller erreicht werden soll, besteht ausschließlich darin, daß die sich im Innenraum befindliche Medizin entnommen und von dem Kind aufgenommen werden kann.

Es ist daher Aufgabe der Erfindung, in Abwendung von der reparativen Zahnheilkunde bei auftretenden Kariesfällen für die Primär-Prävention und der Kausaltherapie von Karies eine Vorrichtung,für Säuglinge zu schaffen, die eine posteruptive Optimierung der Schmelzmaturation und der Pellikelreifung der Milchzähne und eine Kolonisationshemmung von Mutans streptokokken an diesen Zähnen ermöglicht und mit,der in Anpassung an die Entwicklung der Milchzähne die zu Karies führenden Krankheitserreger zur Vermeidung einer Dauerinfektion wirksam bekämpft werden.

Diese Aufgabe wird bei einer Vorrichtung der eingangs genannten Art durch die in Anspruch 1 angegebenen Merkmale gelöst.

Mit einer derart erfindungsgemäß ausgebildeten Vorrichtung ist ein Schnuller für Säuglinge geschaffen, der aufgrund seiner Ausgestaltung eine optimale Abgabe der im Innenraum des Mundstückes der Vorrichtung vorgesehenen Wirksubstanz, und zwar jeweils in Anpassung an die Milchzahnentwicklung des Kindes, ermöglicht. Durch den Speichel des Kindes werden geringe Spuren der Wirksubstanz gelöst und in die Mundhöhle transportiert, so daß in der Mundhöhle befindliche Infektionserreger vernichtet werden. Ferner wird die vom Speichel des Kindes aufgenommene Wirksubstanz in die Mundhöhle transportiert, wobei die Wirksubstanz zu einer Prävention beiträgt, um zu verhindern, daß sich die die Karies hervorrufenden Mikroorganismen und Bakterien, wie Streptococcus mutans in der Mundhöhle ansammeln und niederlassen. Die orale Gesundheit wird dadurch verbessert.Aber mit der Xylit-NaF enthaltenden Vorrichtung wird nicht nur die Kolonisation von Mutans-streptokokken bekämpft, sondern auch die posteruptive - unmittelbare Phase nach dem Durchbruch der Milchzähne - Schmelzmaturation und die Pellikelreifung der Milchzähne günstig beeinflußt und so die Anfälligkeit dieser Zähne an Karies zu erkranken reduziert. Hinzu kommt noch, daß die sogenannten monoklonalen Antikörper gegen Mutans streptokokken mit Hilfe der Vorrichtung verabreicht -werden können. Monoklonale Antikörper spielen eine sehr wichtige Rolle bei der Kariesprävention. Monoklonale Antikörper werden dabei als Suspension, die z.B. mit Xylit-NaF angenehm schmeckend gemacht worden sind, mit der schnullerartigen Vorrichtung verabreicht. Des weiteren können mit der Vorrichtung synthetisch hergestellte Speichelenzyme, die antibakteriell oder bakterienwachstumhemmend wirken, verabreicht werden. Die Wirksubstanz bzw. die Wirksubstanzen werden in Tabletten-, Pulver- oder in Sirup- bzw. in zäher, viskoser Flüssigkeits-Form verabreicht.

Dabei ist besonders varteilhaft, daß das Mundstück der Varrichtung im unteren, im Gebrauchszustand dem Unterkiefer zugekehrten Bereich mit Durchbrechungen versehen ist, so daß die Wirksubstanz vermittels des Speichels in den Bereich der unteren wachsenden Milchzähne gebracht wird, da die unteren Milchzähne zuerst durchbrechen. Mit der Entwicklung der oberen Milchzähne ist es dann erforderlich, daß auch in diesen Bereich Wirkzubstanz gelangt. Aus diesem Grunde sind im oberen, im Gebrauchszustand dem Oberkiefer zugekehrten Bereich des Mundstückes der Vorrichtung vorgestanzte Abschnitte in der Wand des Mundstückes vorgesehen, die durch Anwendung eines leichten Druckes aus der Wandfläche herausdrückbar sind, um die Durchbrechungen freizugeben, so daß größere Mengen an Speichel der Wirksubstanz zugeführt werden, um diese dem gesamten Mundhöhlenraum zuführen zu können. Die Ausbildung der vorgestanzten Abschnitte ist derart, daß durch die Lutsch- und Saugbewegungen des Säuglings die vorgestanzten Abschnitte nicht herausgedrückt werden, sondern erst bei Aufwenden einer größeren Druckkraft, z.B. durch Zusammenrollen, Kneten u.dgl. des Mundstückes, die vorgestanzten Abschnitte aus der Wandfläche des Mundstückes herausgedrückt werden.

Eine intensivere Aufnahme der Wirkzubstanz durch den Speichel wird durch die Ausführungsform nach Anspruch 2 erreicht. Bei dieser ist das Mundstück der Vorrichtung doppelwandig ausgebildet. In dem Zwischenraum zwischen den beiden Wänden ist dann die Wirksubstanz angeordnet.

Durch die Perforationen in der Außenwand wird Speichel in den Zwischenraum gefördert, wo der Speichel Wirksubstanz aufnimmt, der dann die Wirksubstanz in die Mundhöhle transportiert. Diese Ausgestaltung erbringt den Varteil, daß der Speichel nicht erst in den Innenraum des Mundstückes gebracht werden muff, sondern, daß schon bei geringen Lutsch- und Saugbewegungen Speichel, ohne daß dieser lange Wege durchlaufen muß, an die Wirksubstanz gelangt und diese unmittelbar in die Mundhöhle gebracht wird.

Damit die Durchbrechungen im unteren Bereich des Mundstückes bei aufgenommenem Schnuller im unteren Milchzahnbereich zu liegen kommen, ist die Mundscheibe anatomisch so gestaltet, daß ein Verdrehen des Schnullers während der Lutsch- und Saugbewegungen nicht möglich ist. Für das richtige Einsetzen des Schnullers in den Mund des Säuglings durch die Mutter ist die Mundscheibe gekennzeichnet.

Es wird weiter vorgeschlagen, daß das Aufnahmeorgan integraler Bestandteil der lutschseitigen Partie ist, indem letztere eine innenliegende, hohlförmige Aufnahmekammer für die Wirksubstanz umfaßt mit Perforationen bzw. Durchbrechungen für die Abgabe der Wirksubstanz aus der Kammer an die Mundhöhle.

Die Druchbrechungen werden vorzugsweise derart gewählt, daß die Wirksubstanz nur langsam, d.h. während einer verlängerten Zeitdauer, aus der Kammer an die Mundhöhle abgegeben werden kann. Die Abgabe der Wirksubstanz kann auch derart erfolgen, daß einerseits Speichel aus der Mundhöhle durch die Durchbrechungen in die innenliegende Aufnahme eindringt und dort Wirksubstanz aufnimmt, um dann durch die Durchbrechungen aus der Kammer wieder in die Mundhöhle zu gelangen.

Weiter wird vorgeschlagen, daß die lutschseitige Partie, nämlich das Mundstück, beispielsweise steckbar oder schraubbar mit der Mundsperre lösbar verbunden ist. Durch diese Ausgestaltung der lutschseitigen Partie kann sie von der Mundsperre beispielsweise abgeschraubt werden, damit beispielsweise der innenliegenden, hohlförmigen Aufnahmekammer weitere Wirksubstanz zugeführt werden kann.

Damit beispielsweise ein Kleinkind das Lutschen als angenehm empfindet, ist das Mundstück vorzugsweise aus einem gummiartigen oder einem wenigstens nahezu flexiblen, kunststoffartigen Material hergestellt. Dabei ist allerdings wichtig, daß das gewählte Material heißwasserbeständig respektive sterilisationsfähig ist.

Um feststellen zu können, ob in dem Mundstück bzw. in der darin innenliegend ausgebildeten, hohlförmigen Aufnahmekammer weiterhin Wirksubstanz vorhanden ist, wird weiter vargeschlagen, das Mundstück transparent auszubilden.

Nach einer weiteren erfindungsgemäßen Ausführungsform wird vorgeschlagen, daß die Wirksubstanz,beispielsweise ähnlich einem sog. Schleckstengel, integraler Bestandteil des Mundstückes ist. Bei dieser vorgeschlagenen Ausführungsvariante ist mindestens das Mundstück im Sinne eines Wegwerfartikels vorgesehenen, das jeweils nach einmaligem Gebrauch vollständ ersetzt wird. Möglich ist auch, daß die ganze Vorrichtung ein Wegwerfartikel ist.

Die erfindungsgemäße Vorrichtung eignet sich insbesondere für die Verabreichung von Xylit oder von Xylit/Fluorid-oder Xylit ( > 50Gew%)/Sorbit/Fluorid-Komtinationen, aber auch für die "Langzeit-Verabreichung" eines Medikamentes.

Die Vorrichtung eignet sich aber grundsätzlich für die Mundverabreichung einer Wirksubstanz über eine längere Zeit bei all denjenigen Personen, wo eine akute Gefahr des Verschluckens oder Erstickens bei der Abgabe von Kaugummis, Bonbons usw. besteht. Neben Kleinkindern können dies insbesondere auch ältere Personen sein, die nicht mehr bewußt einen Kaugummi oder eine Lutschtablette im Mund über eine längere Zeit zurückbehalten können.

Weitere vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen
Fig. 1 in einer schaubildlichen Ansicht die schnullerartig ausgebildete Vorrichtung zum oralen Verabreichen einer Wirksubstanz,
Fig. 2 eine schaubildliche Explosionsdarstellung der Vorrichtung gemäß Fig.1,
Fig. 3 eine Ansicht von unten auf die Vorrichtung,
Fig. 4 eine Ansicht von oben auf die Vorrichtung,
Fig. 5 einen senkrechten Längsschnitt gemäß Linie V-V in Fig. 1.
Fig. 6 in einer Seitenansicht eine weitere Ausführungsform der schnullerartigen Vorrichtung mit Verstärkungen des stielförmigen Endes des Mundstückes,
Fig. 7 in einer Seitenansicht die Wirkungsweise der Vorrichtung in Verbindung mit einem unteren Milchzahn und
Fig. 8 in einer Seitenansicht die Wirkungsweise der Vorrichtung in Verbindung mit einem oberen Milchzahn.

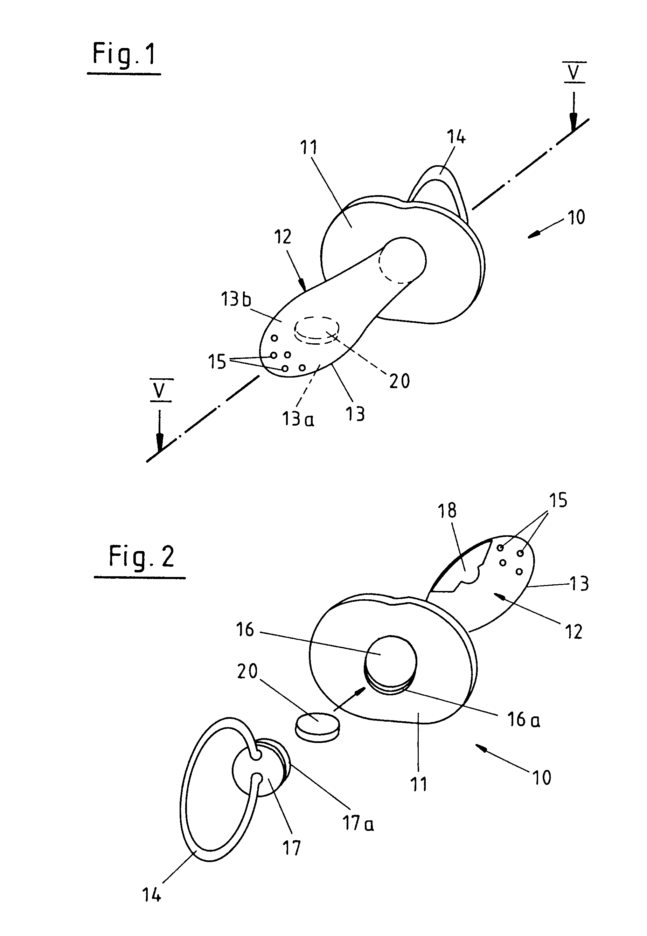

Die in Fig.1 dargestellte und mit 10 bezeichnete Vorrichtung zum oralen Verabreichen einer Wirksubstanz ist als Schnuller für Säuglinge ausgebildet und besteht aus einer Mundscheibe 11 und einem an dieser fest oder lösbar befestigten Mundstück 12, welches den Saug- und Lutschteil der Vorrichtung 10 darstellt. Die Mundscheibe 11 stellt gleichzeitig eine Mundsperre dar und kann anatomisch geformt sein. Diese Mundscheibe 11 besteht aus Kunststoffen oder anderen geeigneten Werkstoffen. Auf der dem Mundstück 12 abgekehrten Seite trägt das Mundstück 12 einen Ring 14, um die Vorrichtung 10 vermittels eines in der Zeichnung nicht dargestellten Bandes in der Nähe des Säuglings befestigen zu können. Das Mundstück 12 der Vorrichtung 10 besteht ebenfalls aus Gummi oder gummiartigen Kunststoffen; dieser Lutsch- und Saugteil der Vorrichtung 10 ist flexibel. Auch glasklare oder transparente Kunststoffe können zur Herstellung des Mundstückes 12 herangezogen werden. Die das Mundstück 12 bildende Wand ist mit 13 bezeichnet,der vom Mundstück 12 umschlossene Innenraum mit 18.

In dem Innenraum 18 des Mundstückes 12 ist eine Wirksubstanz in Pulver-,Sirup- oder Tablettenform 20 angeordnet.Als Wirksubstanz kommt Xylit, monoklonale Antikörper oder aus einer Kombination von Xylit und Fluoriden oder Xylit,Fluoriden und Sorbit zur Anwendung. Als Fluoride kommen insbesondere Natriumfluorid zum Einsatz. Vermittels einer derartigen Wirksubstanz wird ein Kariesbefall bei Säuglingen verhindert. Durch den Speichel mit eingelagerter Wirksubstanz ist ein Verweilen der Wirksubstanz in der Mundhöhle gewährleistet, so daß die Karies bildenden Bakterien in der Mundhöhle wirksam bekämpft werden können.

Des weiteren weist das Mundstück 12 in seinem,im unteren Bereich liegenden Wandabschnitt 13a eine Anzahl von Durchbrechungen 15 auf, die kleinste Abmessungen aufweisen, so daß Speichel während des Saug- und Lutschvorganges in den Innenraum 18 gelangen kann. Dadurch, daß die Mundscheibe 11 anatomisch so geformt ist, daß sich die Vorrichtung 10, während sich diese im Mund des Säuglings befindet, durch die Zungenbewegung nicht verdrehen kann, verbleiben die Durchbrechungen 15 bei sachgemäßer Einführung der Vorrichtung 10 in den Mund des Säuglings immer im unteren Bereich und sind somit den im Unterkiefer angeordneten Milchzähnen zugekehrt, die sich zuerst im Unterkiefer und danach im Oberkiefer entwickeln. Auf diese Weise wird die Wirksubstanz vermittels des Speichels zunsächst direkt in den Bereich der unteren Milchzähne gebracht.

Neben diesen Durchbrechungen 15 im unteren Bereich des Lutsch- und Saugteils der Vorrichtung 10 weist deren Mundstück eine weitere Anzahl von zunächst verschlossenen Durchbrechungen auf, die in dem im oberen Bereich liegenden Wandabschnitt 13b des Mundstückes 12 vorgesehen sind, um nach dem Durchstoßen der oberen Milchzähne auch Wirksubstanz vermittels des Speichels gezielt in diesen oberen Milchzahnbereich bringen zu können. Diese Durchbrechungen werden gebildet von vorgestanzten, kreisförmigen Abschnitten 115. Werden diese durchgedrückt, dann werden die Durchbrechungen freigegeben (Fig.4). Die Stanzung ist bei 115a angedeutet. Die durchdrückbaren Abschnitte sind mit 115b bezeichnet. Die Ausbildung der vorgestanzten Abschnitte 115 ist derart, daß durch die Lutsch- und Saugbewegungen und durch den dabei auftretenden Druck die Abschnitte 115b nicht aus der Wandfläche des Mundstückes 12 herausgedrückt werden. Erst durch Anwendung eines höheren Druckes sind die Abschnitte 115b herausdrückbar, so daß dann im oberen Wandabschnitt 13b die Durchbrechungen ausbildbar sind. Die Durchbrechungen 15 bzw. 115 können sich dabei bis in das stielförmige Ende 12a des Mundstückes 12 erstrecken.

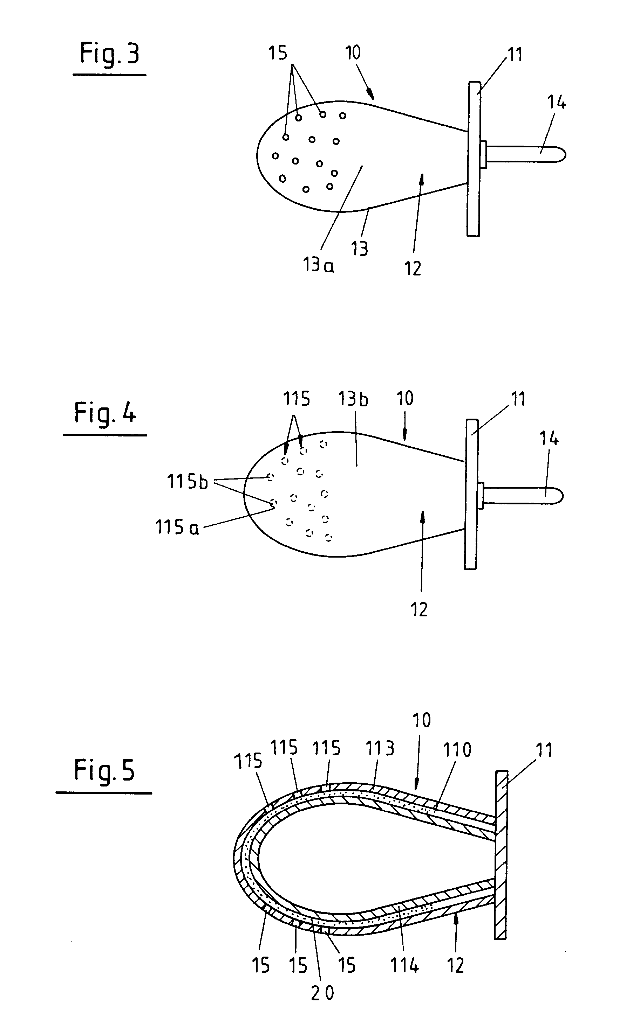

Nach einer weiteren Ausführungsform gemäß Fig.5 ist das Mundstück 12 der Vorrichtung 10 doppelwandig ausgebildet. In dem zwischen den beiden Wänden 113,114, von denen die Wand 113 die Außenwand und die Wand 114 die Innenwand bildet, ausgebildeten Zwischenraum 110 ist die Wirksubstanz 20 angeordnet, die z.B. in Form eines Pulvers oder Granulats vorliegen kann. In der Außenwand 113 sind im unteren Bereich des Mundstückes 12 die Durchbrechungen 15 und im oberen Bereich die durchdrückbaren Abschnitte 115b angeordnet bzw. ausgebildet, wobei auch die Möglichkeit besteht, auch im oberen Bereich des Mundstückes 12 Durchbrechungen 15 entsprechend denen im unteren Bereich vorzusehen. Liegt die Wirksubstanz 20 in Pulverform vor, dann sind die Durchbrechungen 15 so gehalten, daß wohl Speichel beidseitig hindurchtreten kann, jedoch die pulverförmige Wirksubstanz 20 nicht durch die Durchbrechungen hindurchfallen kann.

Die Wand 13 des Mundstückes 12 besteht vorzugsweise aus glasklaren oder transpararenten Werkstoffen, so daß von außen erkennbar ist, ob sich im Innenraum 18 des Mundstückes 12 nach Wirksubstanz 20 befindet. Bei der doppelwandigen Ausführungsform des Mundstückes 12 ist die Außenwand 113 aus glasklaren oder transparenten Werkstoffen gefertigt, da hier die Wirksubstanz in dem Zwischenraum 110 zwischen den beiden Wänden 113,114 angeordnet ist. Die Innenwand 114 besteht dann aus undurchsichtigem Material. Als Werkstoff für das Mundstück 12 kommen geschmacksneutrale, gummielastische Werkstoffe infrage.

Nach der in Fig. 2 gezeigten Ausführungsform weist die Mundscheibe 11 der Vorrichtung 10 eine mittige Durchbrechung 16 mit einem Innengewinde 16a auf. Diese Durchbrechung 16 dient zum Einführen der Wirksubstanz 20 in den Innenraum 18 des Mundstückes 12, das an der Mundscheibe 11, die so bemessen ist, daß sie als Schlucksperre wirkt, im Bereich der Durchbrechung 16 befestigt ist. Der Ring 14 ist an einem plattenförmigen bzw. scheibenförmigen Schraubteil 17 befestigt, das mit einem Außengewinde 17a versehen und so bemessen ist, daß das Schraubteil 17 in die Durchbrechung 16 einschraubbar ist, um diese verschließen zu können.

Für das Einführen einer Wirksubstanz-Tablette 20 oder der Wirksubstanz in Pulver- oder Sirupform mit Xylit, mit einer Xylit/Fluorid- oder einer Xylit/Sorbit/Fluorid-Kombination wird das Schraubteil 17 aus der Durchbrechung 16 herausgeschraubt, so daß der Innenraum 18 nun einseitig offen ist. Die Tablette 10 wird eingeführt und das Schraubteil 17 wiederum in die Durchbrechung 16 eingeschraubt, so daß der Innenraum 18 verschlossen ist.

Die Vorrichtung 10, d.h. der Schnuller, wird so vorbereitet in die Mundhöhle eines Säuglings eingeführt, der aufgrund eines natürlichen Reflexes an dem Mundstück 12 zu saugen beginnt. Infolge des durch die Durchbrechung oder Perforation 15 in den Innenraum 18 eindringenden Speichels wird die Tablette 20 angelöst und Wirksubstanz an den Speichel freigegeben. Durch die Saugbewegung des Säuglings wird der mit Xylit beladene Speichel wiederum durch die Durchbrechungen 15 in die Mundhöhle gesogen, womit nun Xylit an die Mundhöhle freigegeben wird.

Je nach Ausgestaltung der Tablette 20 kann so eine Xylit-Konzentration in der Mundhöhle des Kleinkindes über eine längere Zeit aufrechterhalten werden, womit nun das Streptococcus mutans resp. die Kariesinitiator-Bakterien wirkungsvoll bekämpft werden.

Derjenige Teil der Vorrichtung 10, der die Wirksubstanz 20 in seinem Innenraum aufnimmt, besteht aus wirksubstanzdurchlässigem Material, wie Gummi oder geschmacksneutralen, geeigneten, nicht gesundheitsgefärdenden Kunststoffen.

Sind in dem Mundstück Durchbrechungen vorgesehen, durch die die Wirksubstanz beim Einfließen des Mundspeichels an den Säugling abgegeben werden kann, können andere geeignete Materialien für die Herstellung des Mundstückes verwendet werden.

Des weiteren besteht auch die Möglichkeit, das Mundstück 12 der Vorrichtung 10 als Formkörper auszubilden, der nur aus der Wirksubstanz 20 besteht. Das so ausgebildete Mundstück wird dann lutscherartig verwendet.

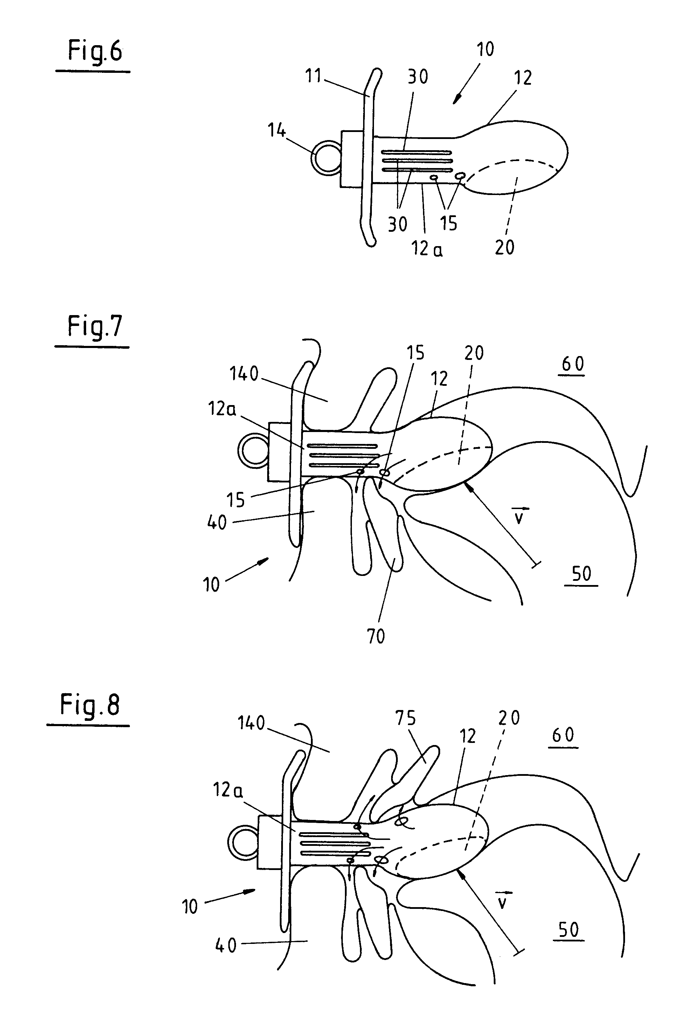

Aus kieferorthopädischen Gründen wird für das Mundstück 12 der Vorrichtung 10 eine anatomische Form bevorzugt, wobei auch andere Formen als geeignet erscheinen. Um eine Beschädigung des stielförmigen Endes 12a des Mundstückes 12 durch Beißen zu verhindern, weist das stielförmige Ende 12a außenwandseitig eine Anzahl von in Vorrichtungslängsrichtung verlaufenden, wulstartigen Verstärkungen 30 auf, die aus gummielastischen Werkstoffen, insbesondere aus Silikonkautschuk, bestehen. Diese Verstärkungen 30 können auch aus dem Material des Mundstückes 12 geformt sein (Fig.6).

In den Fig. 7 und 8 sind mit 40 die Unterlippe, mit 140 die Oberlippe, mit 50 die Zunge, mit 60 der Gaumen der Mundhöhle und mit 70 der untere, erste Milchzahn sowie mit 75 der obere Milchzahn bezeichnet.

Die unteren Durchbrechungen 15 sind in dem Mundstück 12 so angebracht, daß die Wirksubstanz 20 durch den Schluckvorgang und durch den Zungendruck in die Richtung der ersten Milchzähne 70 strömen (Fig.7). Mit dem Durchbruch weiterer Milchzähne, d.h. der oberen Milchzähne 75, werden die Durchbrechungen 115 der neuen Situation angepaßt, so daß die Wirksubstanz die frisch durchbrochenen Milchzahnoberflächen erreichen (Fig.8).

## Patentansprüche

1. Vorrichtung zum oralen Verabreichen einer Wirksubstanz zur prophylaktischen Behandlung von Karies bei Säuglingen, bestehend aus einem Sauger mit einer Mundscheibe (11) und einem an dieser befestigten lutscherartigen Mundstück (12) aus Gummi od.dgl. mit Durchbrechungen (15), durch die in Verbindung mit dem Speichel des Kindes die Wirksubstanz sich auflöst und austritt, wobei die Mundscheibe (11) mit dem Mundstück (12) lösbar verbunden ist, dessen Innenraum (18) als Aufnahmekammer für die Wirksubstanz (20) dient, dadurch gekennzeichnet, daß die Wirksubstanz aus Xylit, monoklonalen Antikörpern gegen Streptococcus mutans und/oder aus Kombinationen von Xylit und Flouriden, wie Natriumfluorid, oder aus Xylit mit Sorbit und Fluoriden besteht und in Pulverform, Sirupform oder in Tablettenform in dem Innenraum (18) angeordnet ist, daß in dem in dem unteren, im Gebrauchszustand dem Unterkiefer zugekehrten Bereich liegenden Wandabschnitt (13a) des Mundstückes (12) eine Anzahl von Durchbrechungen (15) ausgebildet ist und daß in dem in dem oberen, im Gebrauchszustand dem Oberkiefer zugekehrten Bereich liegenden Wandabschnitt (13b) des Mundstückes (12) eine Anzahl von vorgestanzten, kreisförmigen, kleinste Abmessungen aufweisenden, durchdrückbaren und nach erfolgtem Durchdrücken Durchbrechungen (15) bildenden Abschnitten (115) ausgebildet ist.

2. Vorrichtung nach Anspruch 1, dadurch gekennzeichnet, daß das Mundstück (12) der Vorrichtung (10) doppelwandig ausgebildet ist, daß in dem zwischen den beiden Wänden (113,114) ausgebildeten Zwischenraum (110) die Wirksubstanz (20) angeordnet. ist, und daß in der Außenwand (113) die Durchbrechungen (15) und die vorgestanzten Abschnitte (115) angeordnet sind.

3. Vorrichtung nach einem der Ansprüche 1 und 2, dadurch gekennzeichnet, daß in dem im oberen Bereich liegenden Wandabschnitt (13b) Durchbrechungen (15) vorgesehen sind.

4. Vorrichtung nach einem der Ansprüche 1 und 3, dadurch gekennzeichnet, daß die Wand (13) des Mundstückes (12) aus glasklaren oder transparenten Kunststoffen besteht.

5. Vorrichtung nach einem der Ansprüche 1 und 3, dadurch gekennzeichnet, daß die Außenwand (113) und/oder die Innenwand (114) des Mundstückes aus glasklaren oder transpararenten Kunststoffen besteht.

6. Vorrichtung nach einem der Ansprüche 1 bis 5, dadurch gekennzeichnet, daß der stielförmige, freie Endabschnitt (12a) des Mundstückes (12) an seiner Außenwand angeformte und in Vorrichtungslängsrichtung verlaufende Verstärkungswulste (30) aus einem gummielastischen Wirkstoff, wie z.B. Silikonkautschuk, aufweist.

## Revendications

1. Dispositif pour l'administration orale d'un principe actif en vue du traitement prophylactique des caries chez les nourrissons constitué par une tétine avec un disque pour la bouche (11) et un embout du type tétine (15) en caoutchouc ou équivalent, fixé à celui-ci, avec des découpures (15) par lesquelles le principe actif se dissout en relation avec la salive de l'enfant et sort, le disque pour la bouche (11) étant relié de manière amovible à l'embout (12) dont l'intérieur (18) sert de compartiment de logement au principe actif (20), **caractérisé en ce** que le principe actif est constitué par du xylite, des anticorps monoclonaux contre le streptococcus mutans et/ou des combinaisons de xylite et de fluorures, comme le fluorure de sodium, ou de xylite et de sorbitol et de fluorures et qu'il est placé sous forme de poudre, de sirop ou de comprimés dans l'espace intérieur (18), qu'un certain nombre de découpures (15) est configuré dans la section de paroi (13a) de l'embout (12) qui est située dans la zone inférieure tournée, à l'état d'utilisation, vers la mâchoire inférieure et qu'un certain nombre de sections (115) pré-poinçonnées, de forme circulaire, qui présentent les dimensions les plus petites, qui peuvent être enfoncées et qui forment des découpures (15) après avoir été enfoncées est configuré dans la section de paroi (13b) de l'embout (12) qui est située dans la zone supérieure, tournée, à l'état d'utilisation, vers la mâchoire supérieure.

2. Dispositif selon la revendication 1, **caractérisé en ce** que l'embout (12) du dispositif (10) est configuré à double paroi, que le principe actif (20) est placé dans l'espace intermédiaire (110) configuré entre les deux parois (113, 114) et que les découpures (15) et les sections pré-poinçonnées (115) sont placées dans la paroi extérieure (113).

3. Dispositif selon l'une des revendications 1 et 2, **caractérisé en ce** que des découpures (15) sont prévues dans la section de paroi (13b) située dans la zone supérieure.

4. Dispositif selon l'une des revendications 1 et 3, **caractérisé en ce** que la paroi (13) de l'embout (12) est constituée par des matières plastiques claires comme du verre ou transparentes.

5. Dispositif selon l'une des revendications 1 et 3, **caractérisé en ce** que la paroi extérieure (113) et/ou la paroi intérieure (114) de l'embout est constituée par des matières plastiques claires comme du verre ou transparentes.

6. Dispositif selon l'une des revendications 1 à 5, **caractérisé en ce** que la section d'extrémité libre, en forme de manche (12a), de l'embout (12) présente des bourrelets de renforcement (30) moulés sur sa paroi extérieure et allant dans le sens longitudinal du dispositif qui sont en un matériau élastique comme du caoutchouc, comme par exemple en caoutchouc au silicone.

## Claims

1. Device for the oral administration of an active principle for the preventive treatment of caries in babies, comprising a pacifier with a disk-shaped mouth guard (11) and a lollipop-like mouthpiece (12) of rubber or suchlike provided with perforations (15) attached to the same, by means of which the active principle dissolves due to the action of the baby's saliva and issues, the oral disk-shaped mouth guard (11) being detachably connected to the mouthpiece (12), whose interior (18) serves as holding or accommodating space of the active principle (20), characterized in that the active principle comprises xylite, monoclonal antibodies against streptococcus mutans and/or combinations of xylite and fluorides, such as sodium fluoride, or of xylite with sorbitol and fluorides and is disposed in the form of powder, syrup or tablets in the interior (18), in that, in the wall section (13a) of the mouthpiece (12) which, when the device is in use, is located within the bottom area facing the lower jaw, a plurality of perforations (15) is provided and in that, in the wall section (13b) of the mouthpiece (12) which, when the device is in use, is located within the top area facing the upper jaw, a plurality of prepunched, circular sections (115) is constructed that possess the smallest dimensions, can be pressed or forced through and, once the pressing-through has been carried out, form perforations (15).

2. Device according to Claim 1, characterized in that the mouthpiece (12) of the device (10) is constructed so as to be double-walled, in that the active principle (20) is disposed in the interstice (110) formed between the two walls (113,114), and in that the perforations (15) and the prepunched sections (115) are to be found in the outer wall (113).

3. Device according to either of Claim 1or 2, characterized in that perforations (15) are provided in the wall section (13b) located within the top area.

4. Device according to any of Claims 1 and 3, characterized in that the wall (13) of the mouthpiece (12) is comprised of pellucid or highly transparent plastics.

5. Device according to any of Claims 1 and 3, characterized in that the outer wall (113) and/or the inner wall (114) of the mouthpiece (12) is comprised of pellucid or highly transparent plastics.

6. Device according to any of Claims 1 to 5, characterized in that the stem-like free terminal section (12a) of the mouthpiece (12) is provided with reinforcing beads (30) of a rubber-like material, such as e.g. silicon rubber, which are formed onto its outer wall and proceed in the longitudinal direction of the device.
